# EUROPEAN PATENT APPLICATION

(11) **EP 4 438 593 A1**
(43) Date of publication of application: **02.10.2024**
(21) Application number: 23169129.6
(22) Date of filing: 21.04.2023
(51) Int. Cl.: C07C 311/51

(54) **POTASSIUM SALT OF NIMESULIDE AND METHOD FOR OBTAINING POTASSIUM SALT CRYSTALS OF NIMESULIDE**

(30) Priority: 28.03.2023 PL 44422423
(71) Applicant: Uniwersytet Gdanski, 80-309 Gdansk (PL)
(72) Inventor: SIKORSKI, Artur, 83-100 Juszkowo-Pruszcz Gdanski (PL); Rybczynska, Malgorzata, 82-100 Malbork (PL)
(74) Representative: Pawlowska-Bajerska, Justyna

(57) **Abstract**

According to the invention are new compounds potentially pharmaceutically active from the NSAID group - crystals of the potassium salt of nimesulide with the sum formula C₁₃H₁₁N₂O₅SK - shown as formula 1 and the hydrated potassium salt with the sum formula C₃₉H₅₀N₆O₂₃S₃K₂ - shown as formula 2. In the compounds shown, nimesulide is present in the ionised form - the anion - and the function of the counterion is performed by the potassium ion K⁺, which unites the inventions. The structures of the compounds differ only in the number of potassium and nimesulide ions and in the absence or presence of water molecules. The invention is also a method of obtaining.

## Description

The invention refers to new substances: crystals of new potassium salts of nimesulide and a method for obtaining them.

Nimesulide - systematic name: N-(4-nitro-2-phenoxyphenyl)methanesulfonamide is a drug belonging to the group of non-steroidal anti-inflammatory drugs (NSAIDs), available in pharmacies by prescription. It has antipyretic, analgesic and anti-inflammatory properties, as described in Kress, H. G.; Baltov, A.; Basinski, A.; Berghea, F.; Castellsague, J.; Codreanu, C.; Copaciu, E.; Giamberardino, M. A.; Hakl, M.; Hrazdira, L.; Kokavec, M.; Lejčko, J.; Nachtnebl, L.; Stančík, R.; Švec, A.; Tóth, T.; Vlaskovska, M. V.; Woroń, J. (2016) Acute pain: a multifaceted challenge - the role of nimesulide, Current Medical Research and Opinion, 32(1), 23-36.

Like most NSAIDs, the mechanism of action of nimesulide is based on inhibition of the activity of cyclooxygenase, an enzyme involved in the synthesis of prostaglandins from cell membrane lipids. There are two isoforms of this enzyme COX-1 and COX-2. The first enzyme is active under physiological conditions in many tissues. COX-2 is an enzyme whose activity increases rapidly in inflamed tissues. Nimesulide, unlike other NSAIDs, acts preferentially towards COX-2 than COX-1, causing inhibition of prostaglandin synthesis from inflamed tissues, as described in Vane, J. R.; Botting R. M. (1998) Mechanism of Action of Nonsteroidal Anti-Inflammatory Drugs, The American Journal of Medicine, 104, 2-8.

The drug is used orally in the form of granules or tablets, usually at a dose of 200 mg per day. The drug, in gel form for the topical treatment of pain and swelling, is used in a dose of 3 g of gel, 2-3 times daily. The substance is mainly used for the symptomatic treatment of pain for up to 15 days. Nimesulide is a substance classified as a weak acid and exhibiting poor water solubility of 0.01 mg/mL, as shown in Purcaru, S. O.; Ionescu, M.; Raneti, C.; Anuta, V.; Mircioiu, I.; Belu, I. (2010) Study of nimesulide release from solid pharmaceutical formulations in tween 80 solutions, Current Health Sciences Journal, 36(1), 42-9.

Potassium ions are electrolytes with an essential role in the normal functioning of the human body. Potassium is responsible for normal heartbeat and normal muscle function as described in Meneely, G. R.; Battarbee, H. D. (2009). Sodium and Potassium, Nutrition Reviews, 34(8), 225-235. Electrolytes convert very readily to ionic form in an aqueous environment. In addition, just as the name suggests, they cause electrical conduction in solution. Potassium salts show very good solubility in water as published in Kamel, K. S.; Charest, A. F.; Lin S.; Halperin M. (2006). Disorders of Water, Sodium, and Potassium Homeostasis, Clinical Critical Care Medicine, 46, 459-473.

The main obstacle to the use of nimesulide is its poor water solubility and poor solubility in solvents/raw materials typically used in pharmaceutical preparations.

Nimesulide preparations are known to be dispersions of active ingredient particles in a component which, in the case of creams, contains a hydrophilic polymer, an oil substance, a surfactant, an alkali substance and water.

Patent description PL 190603 describes topical preparations of nimesulide in the form of gel systems containing a carboxyvinyl polymer neutralised with an aqueous solution of weak bases, a solvent selected from the group consisting of ethanol, isopropanol, diethylene glycol monoethyl ether, and possibly containing caprylic/capric esters and/or glyceryl(8)OE, stabilising agents and/or preservatives, and having a water content in the range of 40 to 95% by weight. A method of preparation comprising the steps is also described:
- formation of a dispersed aqueous phase containing a carboxyvinyl polymer as a gel-forming agent;
- addition of an alcoholic solvent, selected from the group consisting of ethanol and isopropanol, dispersion of the nimesulide active ingredient, possibly addition of preservatives and stabilizing agents;
- neutralization of the resin with an aqueous solution of a weak base.

From patent description PL 202852 is known a composition for topical application which comprises nimesulide in an amount of 0.1 to 5% by weight per composition, glyceryl monooleate in an amount of 16,5 to 59% by weight per weight of the composition and a non-aqueous solvent in an amount of 25 to 82% by weight per weight of the composition selected from the group consisting of C1-C6-alcohol, N-methylpyrrolidone, glycol or glycol ether, C8-C22 glyceride or ethoxylated glyceride.

The scientific team of the invention deals with nimesulide salt crystals as well as their preparation.

Research to increase the bioavailability of poorly water-soluble drugs is an important aspect in the design of products with therapeutic properties. One method of achieving this is to complex the pharmaceutically active substance with excipients. The creation of new drug forms - with improved properties and, in particular, with the least possible side effects associated with their intake - is possible by designing multicomponent crystals in the form of salts, solvates, polymorphs, or cocrystals (Thakuria, R.; Sarma, B. (2018). Drug-Drug and Drug-Nutraceutical Cocrystal/Salt as Alternative Medicine for Combination Therapy: A Crystal Engineering Approach, Crystals, 8(2), 101, 1-39). Obtaining multicomponent crystals in which at least one of the components is a pharmaceutically active ingredient makes it possible to improve the pharmacodynamic properties of drugs that have a limited range and require constant control of blood concentrations, as well as to minimise the side effects of their use (Blagden, N.; de Matas, M.; Gavan, P. T.; York, P. (2007) Crystal engineering of active pharmaceutical ingredients to improve solubility and dissolution rates, Advanced Drug Delivery Reviews, 59(7), 617-630). therapeutic

From the state of the art described in The Cambridge Structural Database (CSD) to date, it appears that few multi-component crystals involving nimesulide have been known to date. The CSD is a worldwide database containing a collection of all crystal structures of organic and organometallic compounds. Currently, 14 complexes involving nimesulide can be found in the database: four are structures of two polymorphic varieties of nimesulide studied at room temperature and low temperature, one of a nimesulide derivative methylated on the nitrogen atom, and the remaining five, structures of nimesulide complexes with silver as described in: Dupont, L.; Pirotte, B.; Masereel, B.; Delarger, J.; Geczy, J. (1995) Acta Crystallographica, C51, 507-509; Sanphui, P.; Sarma, B.; Nangia, A. (2011) Phase transformation in conformational polymorphs of nimesulide, Journal of phramace, Journal of Pharmaceutical Sciences, 100(6), 2287-2299; Michaux, C.; Charlier, C.; Julemont, F.; Norberg, B.; Dogne, J. M.; Durant, F. (2001) Acta Crystallographica, E57, 1012-1013 Investigations into the activity of certain complexes demonstrate that they possess not only analgesic but also antitumour properties. Nimesulide-silver complexes described in Banti, C. N.; Papatriantafyllopoulou, C.; Manoli, M.; Tasiopoulos, A. J.; Hadjikakou, S. K. (2016) Nimesulide Silver Metallodrugs, Containing the Mitochondriotropic, Triaryl Derivatives of Pnictogen; Anticancer Activity against Human Breast Cancer Cells, Inorganic Chemistry, 55, 17, 8681-8696 show protective properties against breast cancer development. In addition, 4 new cocrystal structures of nimesulide and pyridine derivatives have been reported in 2020 base as described in: Wang, M.; Ma, Y.; Shi, P.; Du, S.; Wu, S.; Gong J. (2021) Similar but Not the Same: Difference in the Ability to Form Cocrystals between Nimesulide and the Pyridine Analogues, Crystal Growth & Design, 21(1), 287-296.

Nimesulide is not present in the ionised form (nimesulide anion) in any of the above structures.

The essence of the invention are new compounds potentially pharmaceutically active from the NSAID group - crystals of the potassium salt of nimesulide with the sum formula C₁₃H₁₁N₂O₅SK - shown as formula 1 and the hydrated potassium salt with the sum formula C₃₉H₅₀N₆O₂₃S₃K₂ - shown as formula 2. In the compounds shown, nimesulide is present in the ionised form - the anion - and the function of the counterion is performed by the potassium ion K⁺, which links the inventions. The structures of the compounds differ only in the number of potassium and nimesulide ions and in the absence or presence of water molecules.

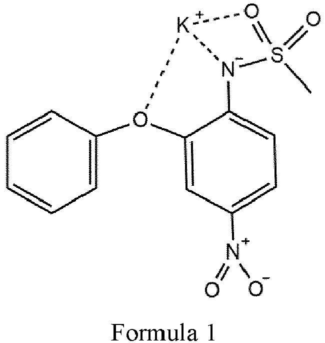

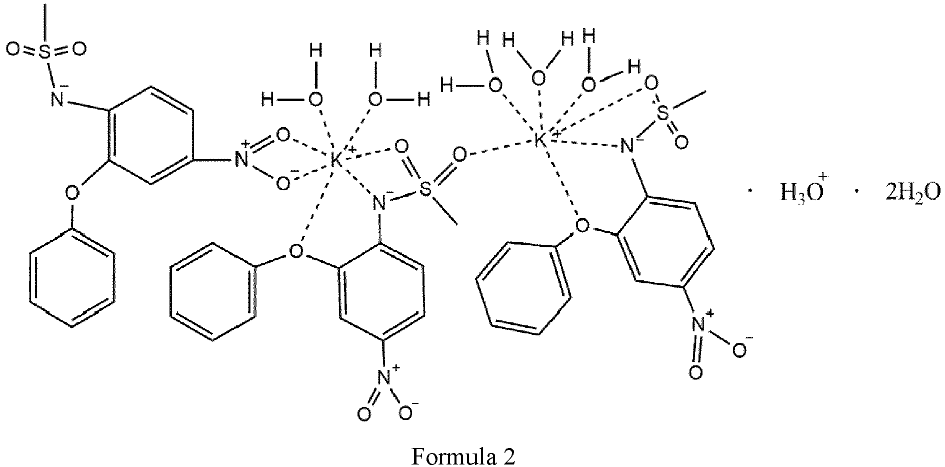

Preferably, the crystals of the potassium salt of nimesulide are in the form of solid crystals, polymorphs - especially I, II or II, solvates or cocrystals or in the form of solutions in various solvents.

The invention may have applications in the pharmaceutical industry as an analgesic, anti-inflammatory and antipyretic, in osteoarthritis and other diseases, as well as in the preparation of drugs with anticancer effects, as confirmed by studies of the substance's properties described in the scientific literature.

The subject matter is method for producing crystals of potassium salts of nimesulide.

The method of producing crystals of potassium salts of nimesulide consists in the fact that the substrates: nimesulide and potassium hydroxide, in the form of powdered solids, are mixed with each other in any % by weight/molar ratio depending on the number of grams of product to be obtained and the yield of the reaction, and then this mixture is dissolved in any solvent or mixture of solvents in such a volume ratio as to dissolve completely mentioned mixture. The reaction mixture is stirred at room temperature until the substrates are completely dissolved. The reaction occurs with an efficiency of not less than 50% when nimesulide and potassium hydroxide are mixed in a molar ratio of 1:1, which is beneficial. The reaction mixture is stirred after the addition of the solvent or solvent mixture until the substrates are completely dissolved at room temperature, e.g. about 1 minute. The resulting solution is then left at room temperature to slowly evaporate in a place out of sunlight for several days until the substance is obtained in crystal form.

Advantages resulting from the invention:
The substances according to the invention have a good solubility in water at room temperature of at least 1.9 mg/mL, which is at least 100 times higher than the water solubility of nimesulide of 0.01 mg/mL, making them have a higher bioavailability than pure nimesulide.

The object of the invention is illustrated in more detail in the examples and drawings, where: Fig. 1 shows the asymmetric part of the elementary cell of a potassium nimesulide salt of formula 1 (polymorph I) with the indicated numbering of the atoms, Fig. 2 shows the packing of ions in the crystal lattice of a potassium nimesulide salt (polymorph I) of formula 1 showing the connection of ions through hydrogen bonds (indicated by a dashed line), Fig. 3 shows the melting temperature diagram of the crystals of the potassium nimesulide salt of formula 1 (polymorph I) made on a BUCHI cryometer, Fig. 4 shows the asymmetric part of the elementary cell of the potassium nimesulide salt of formula 1 (polymorph II) with the numbering of the atoms Fig. 5 shows the packing of ions in the crystal lattice of the potassium nimesulide salt of formula 1 (polymorph II) showing the connection of ions through hydrogen bonds (indicated by the dotted line), Fig. 6 shows the melting temperature diagram of the potassium nimesulide salt of formula 1 (polymorph II) crystals made on a BUCHI cryometer, Fig. 7 shows the asymmetric part of the elementary cell of the potassium nimesulide salt of formula 2 with the indicated atomic numbering Fig. 8 shows the packing of ions in the crystal lattice of the potassium nimesulide salt of formula 2 showing the connection of ions through hydrogen bonds (marked with a dotted line), Fig. 9 shows the melting temperature diagram of crystals of the potassium nimesulide salt of formula 2 made on a BUCHI cryometer.

### Example 1

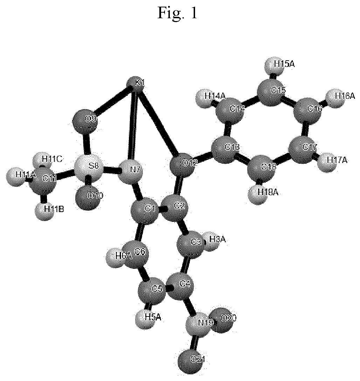

An equal number of grams of substrates were weighed and mixed: 50 mg of nimesulide (0.0162 mmol) and 9 mg of potassium hydroxide (0.0162 mmol). The substrate mixture was then dissolved in ethanol (solvent) in such a volume ratio as to dissolve the above-mentioned mixture completely - at least in 5 cm³ of ethanol - and the whole mixture was stirred for at least 1 minute at room temperature. The beaker with the solution is left at room temperature until the solvent has completely evaporated, in a place out of sunlight. After 5 days, yellow crystals of the product were obtained at the bottom of the beaker. The product obtained are crystals of the potassium salt of nimesulide (formula 1) (polymorph I), as confirmed by XRD X-ray structural analysis and the melting point diagram shown in Table 1 and Fig. 1-3. XRD X-ray structural analysis measurements were carried out at 20°C on a Gemini R Ultra four-circle diffractometer with a Ruby CCD detector, from Oxford Diffraction using radiation with a wavelength of λ_{Cu} =1.54184 Å. Data registration, reduction and analysis were performed using CRYSALIS-CCD and CRYSALIS-RED software. The structure was resolved by direct methods using SHELXS-2013 and refined using SHELXL-17, as shown in Table 1.

**Table 1. Selected crystallographic data for a crystal of the potassium salt of nimesulide (formula 1) (polymorph I).**

| Empirical formula | C₁₃H₁₁N₂O₅SK |
|---|---|
| Molar mass [g/mol] | 346,40 |
| Measurement temperature [K] | 293(2) |
| Wavelength of radiation used [Å] | 1,54184 |
| Crystal system | monoclinic |
| Space group | *P*2₁/n |
| Unit cell parameters | a = 5,4037(3) Å α = 90° |
| | b = 17,1864(8) Å β = 99,044(5)° |
| | c = 16,0256(10) Å γ = 90° |
| Volume of the unit cell [Å³] | 1469.81(14) |
| Number of molecules in an unit cell | 4 |
| Theoretical crystal density [mg/m³ ] | 1,565 |
| Linear absorption coefficient [mm⁻¹] | 4,739 |
| Range of the θ angle [°] | 3,797 - 67,371 |
| Data/restraints/parameters | R1 = 0,0462, wR2 = 0,1084 |
| Final R1 and R2 values (I> 2σ(*I*)) | R1 = 0,0672, wR2 = 0,1203 |

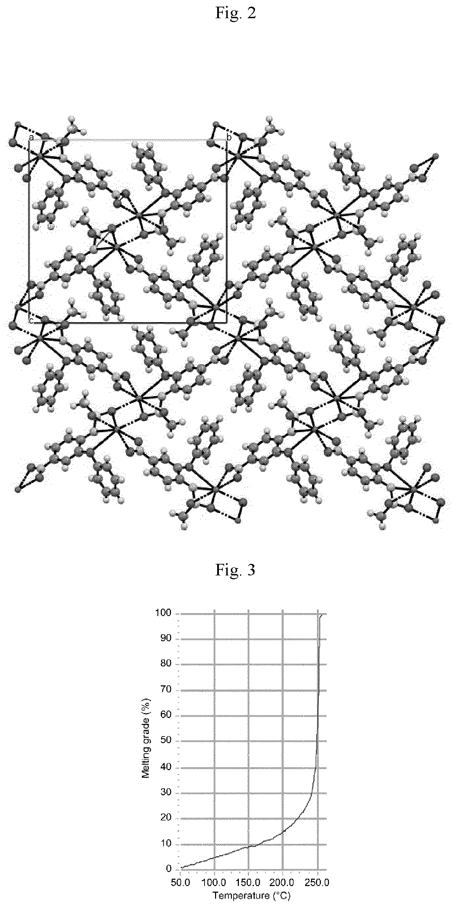

It can be seen from Table 1 that the potassium salt of nimesulide of formula 1 (polymorph I) crystallises in a monoclinic *P*2₁/n space group. The asymmetric part of the unit cell of the crystals of compound obtained contains one nimesulide anion and one potassium anion, as shown in Fig. 1. Analysis of the packing in the crystal lattice shows that the ions are connected through hydrogen bonds, as shown in Fig. 2.

Using the BUCHI apparatus, the melting point of the crystals of the resulting salt was tested, which is 253.7°C, as shown in the melting point diagram in Fig. 3.

Tests carried out to determine the solubility of the potassium salt crystals of nimesulide of formula 1 show that the substance according to the invention has a good solubility in water at room temperature of at least 2.0 mg/mL, which is at least 100 times higher than the solubility of nimesulide in water of 0.01 mg/mL.

### Example 2

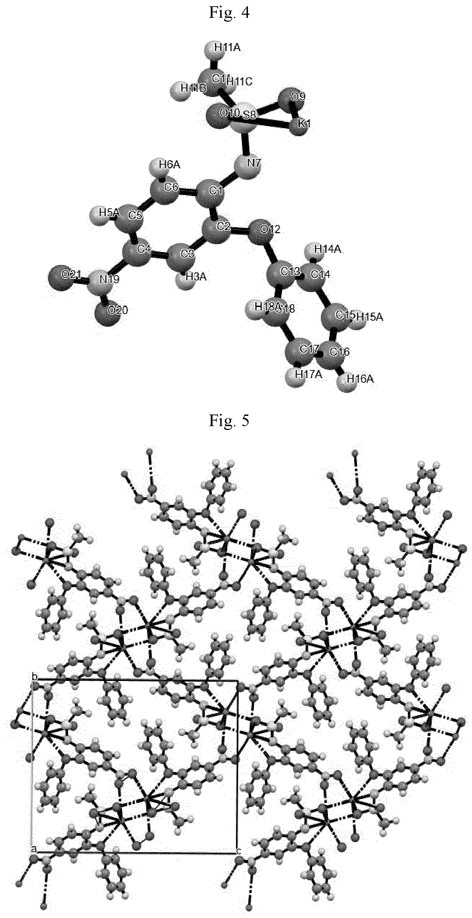

50 mg of nimesulide (0.0162 mmol) was weighed and added to 9 mg of potassium hydroxide (0.0162 mmol). In addition, 9.5 mg of 1,2,3,4-tetrahydro-9-acridinamine hydrochloride (0.00405 mmol) was weighed and added to the system. The substrate mixture was then dissolved in ethanol (solvent) in such a volume ratio as to dissolve the aforementioned mixture completely - at least in 5 cm³ ethanol - and the whole mixture was stirred for at least 1 minute at room temperature. The beaker with the solution is left at room temperature until the solvent has completely evaporated, in a place out of sunlight. After 7 days, yellow crystals of the product were obtained at the bottom of the beaker. The product obtained is crystals of the potassium salt of nimesulide (formula 1) (polymorph II), as confirmed by XRD X-ray structural analysis and the melting point diagram shown in Table 2 and Figs. 4-6. XRD X-ray structural analysis measurements were carried out at 20°C on a Gemini R Ultra four-circle diffractometer with a Ruby CCD detector, from Oxford Diffraction using radiation with a wavelength of λ_{Cu} =1.54184 Å. Data registration, reduction and analysis were performed using CRYSALIS-CCD and CRYSALIS-RED software. The structure was resolved by direct methods using the SHELXS-2013 program and refined using the SHELXL-17 programme, as shown in Table 2.

**Table 2. Selected crystallographic data for a crystal of the potassium salt of nimesulide (formula 1) (polymorph II).**

| Empirical formula | C₁₃H₁₁N₂O₅SK |
|---|---|
| Molar mass [g/mol] | 346,40 |
| Measurement temperature [K] | 293(2) |
| Wavelength of radiation used [Å] | 1,54184 |
| Crystal system | rhombic |
| Space group | *P*2₁2₁2₁ |
| Unit cell parameters | a = 5,53280(12) Å α = 90 ° |
| | b= 14,8587(3) Å β = 90 ° |
| | c = 17,4577(3) Å γ = 90 ° |
| Volume of the unit cell [Å³] | 1435,20(5) |
| Number of molecules in an unit cell | 4 |
| Theoretical crystal density [mg/m³ ] | 1.603 |
| Linear absorption coefficient [mm⁻¹] | 4,853 |
| Range of the θ angle [°] | 5,067 - 67,222 |
| Data/restraints/parameters | R1 = 0,0369, wR2 = 0,0881 |
| Final R1 and R2 values (I> 2σ(*I*)) | R1 = 0,0428, wR2 = 0,0926 |

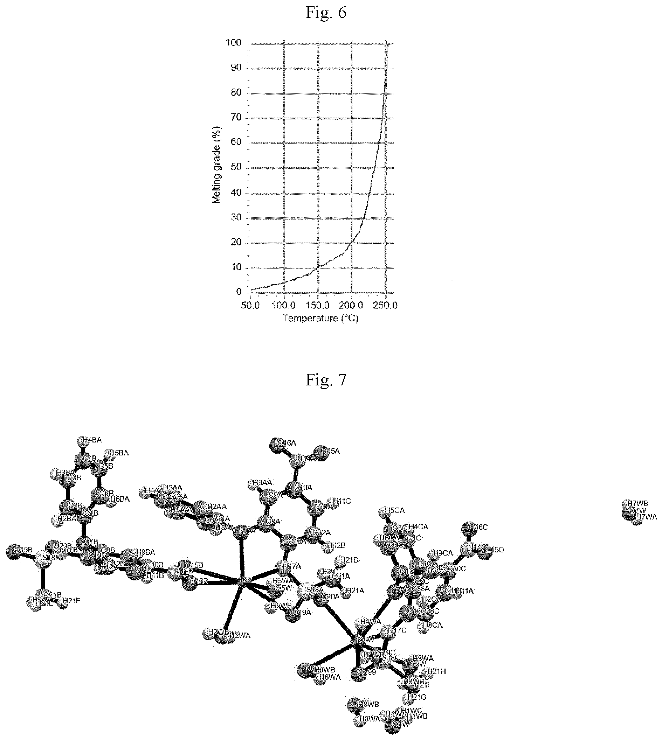

It can be seen from Table 2 that the potassium salt of nimesulide of formula 1 (polymorph II) crystallizes in a orthorhombic *P*2₁2₁2₁ space group. The asymmetric part of the unit cell of the crystals of compound obtained contains one nimesulide anion and one potassium anion, as shown in Fig. 4. Analysis of the packing in the crystal lattice shows that the ions are connected through hydrogen bonds, as shown in Fig. 5. Using the BUCHI apparatus, the melting point of the crystals of the resulting salt was tested, which is 251.8°C, as shown in the melting point diagram in Fig.6.

Tests carried out to determine the solubility of potassium salt crystals of nimesulide of formula 1 (polymorph II) show that the substance according to the invention has a good solubility in water at room temperature of at least 2.1 mg/mL, which is at least 100 times higher than the solubility of nimesulide in water of 0.01 mg/mL.

### Example 3

An equal number of grams of substrates were weighed and mixed: 50 mg of nimesulide (0.0162 mmol) and 9 mg of potassium hydroxide (0.0162 mmol). The substrate mixture was then dissolved in water (solvent) in such a volume ratio as to dissolve the aforementioned mixture completely - at least in 5 cm³ of water - and the whole mixture was stirred for at least 1 minute at room temperature. The beaker with the solution is left at room temperature until the solvent has completely evaporated, in a place out of sunlight. After 14 days, yellow crystals of the product were obtained at the bottom of the beaker. The product obtained is nimesulide potassium salt crystals (formula 2), as confirmed by XRD X-ray structural analysis and the melting point diagram shown in Table 3 and Fig. 7-9. XRD X-ray structural analysis measurements were carried out at 20°C on a Gemini R Ultra four-circle diffractometer with Ruby CCD detector, from Oxford Diffraction using radiation at λ_{Mο} = 0.71073Å. Data registration, reduction and analysis were performed using CRYSALIS-CCD and CRYSALIS-RED software. The structure was resolved by direct methods using SHELXS-2013 and refined using SHELXL-17, as shown in Table 3.

**Table 3. Selected crystallographic data for the nimesulide potassium salt crystal (formula 2).**

| Empirical formula | C₃₉H₅₀N₆O₂₃S₃K₂ |
|---|---|
| Molar mass [g/mol] | 1145,23 |
| Measurement temperature [K] | 293(2) |
| Wavelength of radiation used [Å] | 0,71073 |
| Crystal system | triclinic |
| Space group | *P*-1 |
| Unit cell parameters | a = 8,1110(3) Å α = 114,544(7)° |
| | b=17,6040(13) Å β = 98,888(4)° |
| | c = 20,1459(14) Å γ = 94,562(4)° |
| Volume of the unit cell [Å³] | 2552,0(3) |
| Number of molecules in an unit cell | 2 |
| Theoretical crystal density [mg/m³ ] | 1,490 |
| Linear absorption coefficient [mm⁻¹] | 0,395 |
| Range of the θ angle [°] | 3,26 - 25,00 |
| Data/restraints/parameters | R₁ = 0,0684, wR₂ = 0,1588 |
| Final R1 and R2 values (I > 2σ(*I*)) | R₁ = 0,1573, wR₂ = 0,2098 |

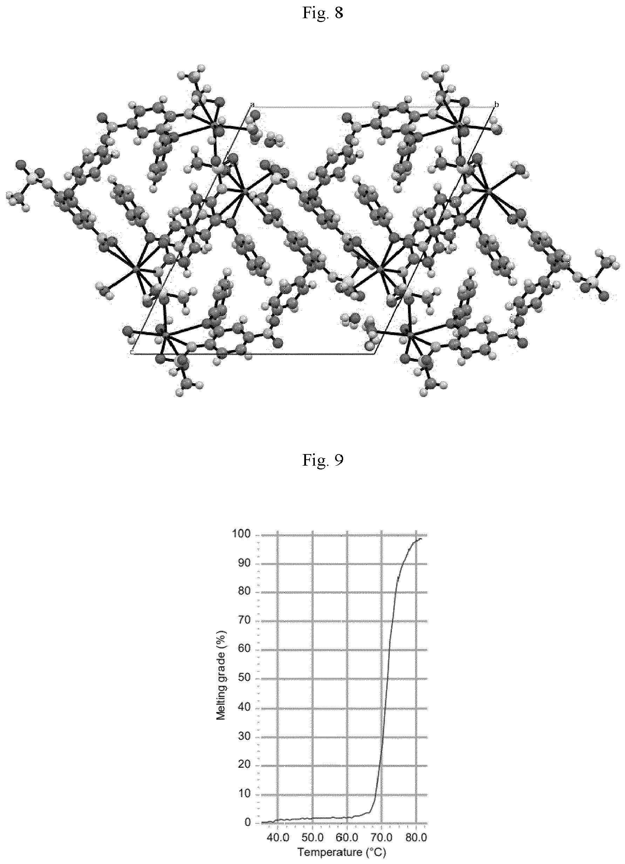

From Table 3, it can be seen that the potassium salt of nimesulide of formula 1 (polymorph II) crystallizes in the triclinic *P*-1 space group. The asymmetric part of the unit cell of the crystals of compound obtained contains three nimesulide anions, two potassium cations, one hydronium ion and seven water molecules, as shown in Fig. 7. Analysis of the packing in the crystal lattice shows that the ions are connected through hydrogen bonds, as shown in Fig. 8.

Using the BUCHI apparatus, the melting point of the crystals of the resulting salt was tested, which is 79.2°C, as shown in the melting point diagram in Fig. 9.

Tests carried out to determine the solubility of the potassium salt crystals of nimesulide of formula 2 show that the substance according to the invention has a good solubility in water at room temperature of at least 1.9 mg/mL, which is at least 100 times higher than the solubility of nimesulide in water of 0.01 mg/mL.

## Claims

1. Potassium salt of nimesulide with the sum formula C₁₃H₁₁N₂O₅SK with formula 1: 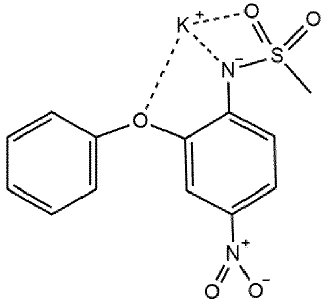 where nimesulide occurs in an ionised form.

2. Potassium salt of nimesulide according to claim 1, wherein it is present in the form of solid crystals, polymorphs, solvates or cocrystals or in the form of solutions in various solvents.

3. Potassium salt of nimesulide with the sum formula C₃₉H₅₀N₆O₂₃S₃K₂ with formula 2: 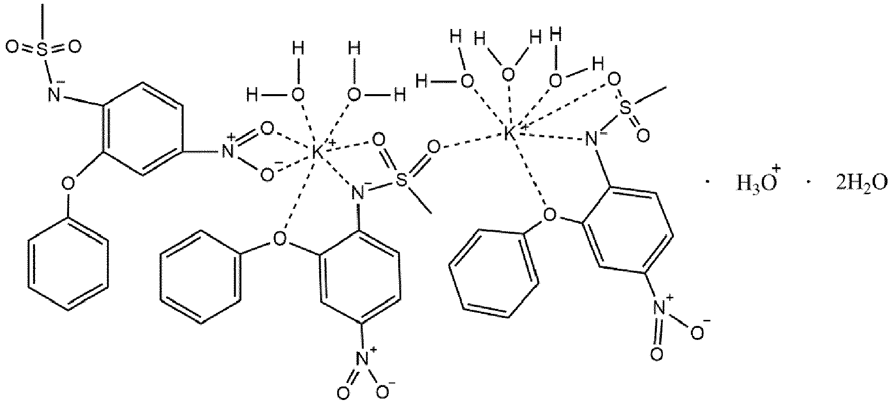 where nimesulide occurs in an ionised form.

4. Potassium salt of nimesulide according to claim 3, wherein it present as a crystalline solid, its polymorphs, solvates or cocrystals or as solutions in various solvents.

5. A method for obtaining potassium salt crystals of nimesulide of formula 1, **characterized in that** it comprises preparation of an equimolar mixture of nimesulide and a compound constituting a source of potassium ions, preferably hydroxide, and the substrates are mixed together at different molar ratios depending on the number of grams of product to be obtained and the yield, preferably in an equimolar ratio, and then dissolved in a known solvent or mixture of solvents in an amount until the mixture is completely dissolved, and the solution thus obtained is left until the solvent is completely evaporated and the compound crystalizes.

6. Method for obtaining potassium salt crystals of the nimesulide of formula 2, **characterized in that** it comprises preparation of a mixture of nimesulide and a compound constituting a source of potassium ions, preferably hydroxide, and the substrates are mixed with each other at different molar ratios depending on the number of grams of product to be obtained and the yield, preferably in an equimolar ratio, and then the mixture is dissolved in a known solvent or mixture of solvents in an amount until the mixture is completely dissolved, and the solution thus obtained is left until the solvent is completely evaporated and the compound crystallizes.
